# EUROPEAN PATENT APPLICATION

(11) **EP 4 725 322 A1**
(43) Date of publication of application: **15.04.2026**
(21) Application number: 25207835.7
(22) Date of filing: 09.10.2025
(51) Int. Cl.: A23L 33/135, A23L 33/175, A61K 31/198, A61K 35/747, A61P 25/16

(54) **PROBIOTIC MIXTURE FORMULA FOR USE IN A MEDICAMENT FOR DELAYING THE PROGRESSION OF PARKINSON'S DISEASE**

(30) Priority: 10.10.2024 US 202463705596 P
(71) Applicant: Taipei Medical University, Taipei City 110 (TW)
(72) Inventor: HUANG, HUI-YU, Taipei City (TW)
(74) Representative: Cabinet Chaillot

(57) **Abstract**

The present invention relates to a probiotic cocktail medical food composition and its use in delaying the progression of Parkinson's disease. The composition comprises: (a) at least one probiotic with neuroprotective or metabolic regulatory functions; (b) a functional amino acid combination comprising four or more essential amino acids. The composition can be formulated as a medical food product for improving or alleviating pathological conditions associated with Parkinson's disease, including motor dysfunction, cognitive decline, inflammation, and mitochondrial impairment. It is suitable for daily nutritional supplementation and disease progression delay in patients with Parkinson's disease or individuals at high risk.

## Description

### REFERENCE TO RELATED APPLICATIONS

The present application is based on, claims priority to, and incorporates herein by reference in its entirety for all purposes, U.S. Provisional Application Ser. No. 63/705,596, filed Oct. 10, 2024.

### FIELD OF THE INVENTION

The present invention relates to a nutritional composition having neuroprotective functions, in particular a probiotic cocktail medical food composition comprising probiotics and a functional amino acid combination, and use thereof for delaying the progression of Parkinson's disease. The present invention belongs to the technical field of medical foods and nutritional compositions for the prevention and improvement of neurodegenerative diseases.

### BACKGROUND OF THE INVENTION

Parkinson's disease (PD) is a degenerative disease of the central nervous system, whose primary pathological features are the gradual degeneration and loss of dopaminergic neurons. Common clinical symptoms include bradykinesia, muscle rigidity, resting tremor, and postural instability, and may be accompanied by cognitive dysfunction and emotional abnormalities. Current treatments primarily rely on medication to control symptoms, most commonly with levodopa (L-DOPA) replacement therapy. However, long-term use is prone to fluctuations in efficacy and side effects such as dyskinesia, and cannot fundamentally delay the progression of neurodegeneration. Therefore, developing nutritional intervention strategies with preventive and disease-modifying potential has become an important and urgent direction for adjuvant treatment.

Recent studies have shown that gut microbiota and intestinal immune function are closely related to the development of PD. Some probiotic strains have potential neuroprotective effects, including regulating neuroinflammation, combating oxidative stress, and maintaining the intestinal barrier function. Probiotics can indirectly contribute to the stability of the nervous system by influencing the generation of short-chain fatty acids and immune metabolism. However, most of the existing technologies focus on the benefits of a single probiotic strain on gut health, and specific evidence for applications in PD is still lacking.

Furthermore, essential amino acids, due to their involvement in important physiological processes such as neurotransmitter synthesis, energy metabolism, and muscle protein maintenance, have potential physiological functions such as neuromodulation, anti-inflammation, and mitochondrial function support. In particular, compositions comprising multiple essential amino acids may provide multiple metabolic support pathways and thus have potential to be developed into nutritional formulas for disease interventions, as they can provide multiple metabolic support pathways. While existing studies have preliminarily explored the benefits of these amino acids on neurological functions, they have mostly been presented as general nutritional supplements, without compositions designed for specific disease models or functional validation. Especially for chronic neurodegenerative diseases such as Parkinson's disease, systematic research on specific essential amino acid compositions remains lacking, which is a technical gap.

Conventional medicaments or nutritional approaches often target a single target or mechanism of action, so that they have limited efficacy in controlling the course of chronic neurodegenerative diseases with multiple pathogenic mechanisms, e.g., Parkinson's disease (PD). Recent studies have shown that combining many ingredients with distinct physiological functions and pathways of action, i.e., often referred to as "cocktail therapy," can simultaneously modulate multiple pathological mechanisms, thereby enhancing the overall improvement effect and potential to delay disease progression. This strategy has been applied in various fields, including antiviral treatment, cancer treatment, and treatment of metabolic diseases. However, nutritional cocktail compositions designed for Parkinson's disease are still rare, in particular, there is a lack of a complete intervention regimens that targets clear indications, are applicable as medical foods, and are validated in animal models for the multiple mechanisms of action. Further development is needed.

The prior art mostly focuses on combining probiotics with Chinese herbal medicines, coenzymes, or other nutrients for general health purposes such as improving physical strength, boosting metabolism, or enhancing cardiovascular functions, while providing few applications with clear indications for neurodegenerative diseases like Parkinson's disease. Although some studies and technologies have mentioned that probiotics might affect the central nervous system by regulating the vagus nerve or intestinal functions, these combinations do not involve tryptophan and branched-chain amino acids in most cases. There is also a lack of specific data demonstrating the verification of systematic efficacy using animal models of Parkinson's disease, and there is no clear evidence supporting that the combinations can delay the disease progression. As can be seen, nutritional cocktail formulas for PD still belong to technical needs that have not been met. The composition proposed in the present invention possesses clear inventiveness and distinguishing applications.

Therefore, the present invention provides a probiotic cocktail medical food composition (hereinafter referred to as "the composition" , "the probiotic mixture formula", or "the cocktail composition") that combines at least one probiotic having neuroprotective or metabolic regulation functions with a functional amino acid combination. Through a synergy between the two components, the composition modulates Parkinson's disease-related pathological mechanisms in multiple aspects, including improving motor and cognitive behavioral impairments, protecting dopaminergic neurons, reducing neuroinflammation and oxidative stress, restoring mitochondrial functions in the brain and muscles, and remodeling the gut microbiota composition. The relevant efficacies have been validated at multiple levels in 6-OHDA-induced animal models of PD, demonstrating that the probiotic mixture formula has potential to delay PD progression and is applicable as a nutritional intervention formula for long-term intake.

### SUMMARY OF THE INVENTION

The main purpose of the present invention is to provide a probiotic mixture formula designed for the progression of Parkinson's disease. The composition comprises at least one probiotic with neuroprotective or metabolic regulation functions and a functional amino acid combination. Through a synergy between the two components, the composition modulates Parkinson's disease-related pathological mechanisms at multiple levels, thereby improving motor and non-motor symptoms, protecting dopaminergic neurons, reducing neuroinflammation and oxidative stress, enhancing mitochondrial function, and remodeling the gut microbiota. The probiotic mixture formula is suitable as a nutritional intervention solution with disease-modifying potential and is useful as a daily supplement for Parkinson's disease patients or individuals at high-risk of Parkinson's disease , providing a safe adjuvant therapy option with multi-target regulatory capabilities for long-term use.

For the aforementioned purpose, the present invention provides a composition comprising (a) at least one probiotic with neuroprotective or metabolic regulation functions; and (b) a functional amino acid combination comprising four or more essential amino acids.

For the aforementioned purpose, the probiotic with neuroprotective or metabolic regulation functions is selected from the group consisting of *Lactobacillus, Lactococcus, Streptococcus, Bifidobacterium, Pediococcus,* and *Enterococcus.*

For the aforementioned purpose, the *Lactobacillus* is selected from the group consisting of *L. acidophilus, L. antri, L. brevis, L. casei, L. coleohominis, L. crispatus, L. curvatus, L. fermentum, L. gasseri, L. johnsonii, L. mucosae, L. pentosus, L. plantarum, L. reuteri, L. rhamnosus, L. sakei, L. salivarius, L. paracasei, L. kisonensis, L. paralimentarius, L. perolens, L. apis, L. ghanensis, L. dextrinicus, L. shenzenensis* and *L. harbinensis.*

For the aforementioned purpose, the probiotic is a viable bacterium, a dead bacterium, a bacterium sterilized at low temperatures, a lysate, a bacterial supernatant, a cell wall extract, an extracellular polysaccharide, or an immunostimulatory component thereof.

For the aforementioned purpose, the essential amino acids are selected from the group consisting of leucine, isoleucine, valine, lysine, methionine, phenylalanine, tryptophan, threonine and histidine.

For the aforementioned purpose, the probiotic mixture formula is a pharmaceutical composition, a nutritional supplement or a healthcare food.

Fore the aforementioned purpose, the composition is a pharmaceutical composition, a nutritional supplement or a healthcare food.

For the aforementioned purpose, the probiotic mixture formula comprises a pharmaceutically acceptable carrier, excipient, or diluent.

For the aforementioned purpose, the composition comprises a pharmaceutically acceptable carrier, excipient, or diluent.

For the aforementioned purpose, the composition is in the form of a powder, a tablet, a capsule or a beverage.

Another purpose of the present invention is to provide a use of the probiotic mixture formula as described above for the preparation of a medicament for delaying the progression of Parkinson's disease.

For the aforementioned purpose, the present invention provides a composition for use in a medicament for delaying the progression of Parkinson's disease.

For the aforementioned purpose, delaying the progression of Parkinson's disease is to reduce Parkinson's disease-related motor symptoms or/and to reduce Parkinson's disease-related non-motor symptoms.

For the aforementioned purpose, the non-motor symptoms associated with Parkinson's disease that are reduced include cognitive dysfunction, memory impairment, disorientation, mental decline, executive function impairment, irritability, changes in interpersonal behavior, anxiety, mood swings, depression, apathy, sleep disorders, dysautonomia, olfactory disorder, abnormal pain perception, or fatigue.

For the aforementioned purpose, the dysautonomia includes constipation, hypotension, urinary dysfunction, or abnormal sweating.

For the aforementioned purpose, the Parkinson's disease-related motor symptoms that are reduced include tremor, muscle rigidity, bradykinesia, unsteady gait, foot shuffling, abnormal involuntary movements, facial expression disorders, and ocular dysfunction.

For the aforementioned purpose, the abnormal involuntary movements include dyskinesia and dystonia.

For the aforementioned purpose, the facial expression disorders include mask-like facies and facial paralysis.

For the aforementioned purpose, the ocular dysfunction includes apraxia of eyelid opening, blepharospasm, reduction of tear film secretion, and evaporative dry eye caused by non-blinking responses.

For the aforementioned purpose, delaying the progression of Parkinson's disease is achieved by enhancing anti-inflammatory capacity, enhancing antioxidant capacity, restoring mitochondrial metabolic function, regulating gut microbiota, or restoring intestinal microecological balance.

For the aforementioned purpose, the probiotic mixture formula is used in daily nutritional supplementation for Parkinson's disease patients or Parkinson's disease high-risk groups to delay the progression of the disease.

In summary, the probiotic mixture formula disclosed in the present invention is useful for the preparation of a nutritional combination formulation for delaying the progression of Parkinson's disease, and also in daily nutritional supplementation for Parkinson's disease patients or Parkinson's disease high-risk groups. Its application benefits include improving motor dysfunction, enhancing cognitive performance, protecting neurons, reducing neuroinflammatory response and anti-oxidative stress, and restoring the balance between mitochondrial energy metabolism and gut microbiota, thereby exhibiting the systemic disease-modifying potential. It possesses both technical inventiveness and clinical application values. It is a multi-mechanism, multi-target, safe and accessible non-drug nutritional intervention strategy that is expected to serve as an adjuvant solution for early intervention and delaying the progression of Parkinson's disease. It is also potentially expanded to be applied in the prevention and supportive treatment of other neurodegenerative diseases.

### BRIEF DESCRIPTION OF THE DRAWINGS

The techniques of present invention would be more understandable from the detailed description given herein below and the accompanying figures are provided for better illustration, and thus description and figures are not limitative for present invention, and wherein:
FIG. 1 is timeline of the PD-induction protocol in the animal experiment.
FIG. 2 shows behavioral assessments of the motor function in Parkinson's disease rat model: (A) Apomorphine rotation test at week 2; (B) Apomorphine rotation test at week 10; (C) Rotarod test; (D) Grip strength test.
FIG. 3 shows spatial learning and memory test in the Morris water maze test: (A) swimming trajectory; (B) Statistics of escape latency; (C) Time spent in the target quadrant and total swimming distance.
FIG. 4 shows immunohistochemical staining and quantification of TH-positive dopaminergic neurons in the substantia nigra pars compacta (SNpc).
FIG. 5 shows the expression of pro-inflammation and anti-oxidant-related genes in the striatum: (A) *Tnf-α;* (B) *IL-6; (C) Gpx; (D) Cat.*
FIG. 6 shows the mitochondrial function in the striatum of brain: (A) OCR; (B) ECAR; (C) *Sirt1* gene expression; (D) *Pgc-1α* gene expression.
FIG. 7 shows the mitochondrial function in the soleus muscle: (A) OCR; (B) ECAR; (C) *Sirt1* gene expression; (D) *Pgc-1α* gene expression.
FIG. 8 shows the relative abundance of gut microbiota.
FIG. 9 shows the LEfSe analysis results.

### DETAILED DESCRIPTION OF THE INVENTION

All technical and scientific terms used in this specification have the meanings commonly understood by those ordinarily skilled in the art unless otherwise defined. The materials used in the present invention are commercially, easily available unless otherwise specified.

The term "a" or "an" does not exclude a plurality, that is to say, unless the context clearly indicates or requires otherwise, the singular forms "a", "an" and "the" should be understood to include plural referents. In other words, unless otherwise expressly stated or the context of reference clearly implies opposite meanings, references to a singular characteristic or limitation throughout the present invention shall include the corresponding a plurality of characteristics or limitations and *vice versa.* Therefore, unless otherwise defined, the terms "a", "an" and "the" have the same meaning as "at least one" or "one or more". For example, reference to "a component" includes mixtures of many components, etc.

The term "about" or "approximately" can compensate for the variability allowed by the food or pharmaceutical industry and inherent in foods or pharmaceutical products, such as differences in content due to manufacturing variations and/or product degradation over time. This term allows any variation in the actual food or drug to consider the biological properties of a product under evaluation in a subject equivalent to the enumerated advantages of the claimed product.

The term "composition" refers to a composition of any form, into which many specific components may be optionally incorporated in conjunction with any further components.

The term "comprise" and similar contexts should be interpreted in an open and inclusive manner as "including but not limited to".

The terms "an embodiment", "one embodiment", "a specific embodiment", etc., indicate a unique feature, attribute or characteristic, or a group or set of unique features, attributes or characteristics combined with individual contexts to present in at least one embodiment of the present invention. Such contexts anywhere in this patent specification do not necessarily refer to the same embodiment. Furthermore, the particular features, attributes or characteristics may be combined in any suitable manner in one or more embodiments.

The term "prevention" refers to preventing a disease, condition, or symptom, as well as preventing further growth and spread of recurrence or progression after the initial improvement or the initial elimination of the cause of the disease, condition, or symptom.

The term "treatment" refers to a therapeutic intervention that can effectively treat a disease, condition or symptom; however, treatment may also refer to improvement, amelioration, control, controlling progression, preventing progression, preventing recurrence, and so on.

The term "probiotic" refers to a microorganism containing viable microbes, that is, capable of reproduction. Probiotic is widely recognized in the state of the art at the time of the patent application as a microorganism that exerts beneficial effects on the host's health when administered in a proper amount.

The term "probiotic mixture formula" refers to a composite medical food comprising at least one probiotic in combination with nutrients having specific physiological functions (such as essential amino acids). The probiotic mixture formula has many biological activities and can achieve the effect of delaying the progression of Parkinson's disease through various mechanisms (e.g., neuroprotection, anti-inflammation, antioxidation, mitochondrial activation, and regulation of gut microbiota). The probiotic cocktail composition referred to in the present invention is a composite nutritional formula for medical use based on a synergistic multi-component mechanism, applicable as an adjuvant intervention and daily nutritional supplementation for neurodegenerative diseases.

The term "probiotics with neuroprotective or metabolic regulation functions" refers to microbial strains that can regulate neuroinflammation, antioxidant, mitochondrial function, or gut microbiota by direct or indirect mechanisms, thereby exerting the protection or stabilization effects on the nervous system. The probiotic is a viable bacterium, a dead bacterium, a lysate, a bacterial supernatant, an extracellular polysaccharide or a component thereof having immunomodulatory functions.

The term "essential amino acids" refers to α-amino acids that must be obtained through dietary or exogenous supplementation as the human body cannot synthesize on its own, or the synthesis rate is insufficient to meet physiological needs. According to the general nutritional definitions, common essential amino acids are leucine, isoleucine, valine, lysine, methionine, phenylalanine, tryptophan, threonine, and histidine.

The term "delaying the progression of Parkinson's disease" refers to slowing down or inhibiting the progression of Parkinson's disease through some intervention method, including but not limited to improving motor dysfunction, reducing non-motor symptoms, maintaining neuronal integrity or improving quality of life.

The term "Parkinson's disease-related non-motor symptoms" includes, but is not limited to, cognitive dysfunction, memory impairment, disorientation, mood swings, mental decline, executive function impairment, irritability, changes in interpersonal behavior, anxiety, depression, apathy, sleep disorders, dysautonomia (such as constipation, hypotension, urinary dysfunction, abnormal sweating) and hyposmia, abnormal pain perception, and fatigue.

The term "Parkinson's disease-related motor symptoms" includes but is not limited to tremor, muscle rigidity, bradykinesia, unsteady gait, foot shuffling, abnormal involuntary movements (such as dyskinesia and dystonia), facial expression disorders (such as mask-like facies and facial paralysis), ocular dysfunction (such as apraxia of eyelid opening, blepharospasm, reduction of tear film secretion, evaporative dry eye caused by non-blinking responses), etc.

The term "multi-target" refers to an intervention strategy that simultaneously acts on two or more physiological or molecular mechanisms associated with the disease pathology, e.g., modulating neuroinflammatory responses, mitochondrial function, antioxidant capacity, neurotransmission regulation, or balance of gut microbiota. Compared to single-target interventions, multi-target strategies can enhance the overall effectiveness of interventions and facilitate multi-level modulation of the disease progression.

The *Lactobacillus reuteri* Y7 used in this embodiment has been deposited in the Bioresource Collection and Research Center (BCRC) of Taiwan, under the accession number BCRC 911162. This strain has also been deposited in an international depositary of Japan: The International Patent Organism Depositary, National Institute of Technology and Evaluation (IPOD, NITE) (#120, 2-5-8 Kazusakamatari, Kisarazu-shi, Chiba 292-0818, Japan), under the accession number NITE BP-04078.

The present invention relates to a composite nutritional composition designed based on the concept of "cocktail therapy." It combines probiotics having neuroprotective or metabolic regulation functions and functional amino acid combination. Through the synergistic and complementary effects of these components, the composition modulates the physiological and pathological mechanisms associated with the progression of Parkinson's disease. The composition may comprise probiotics from one or more sources, in the form of a viable bacterium, a dead bacterium, a processed bacterium or a derived component thereof, in conjunction with nutrients that potentially modulate neurotransmission, energy metabolism, and anti-inflammation, e.g., essential amino acids. These components are combined in specific proportions to form a palatable dosage form, such as a powder, a tablet, a capsule, and a beverage. The composition can be used as a pharmaceutical composition, nutritional supplement, or a health food for Parkinson's disease patients or high risk groups, and also for daily nutritional supplementation or an adjunctive intervention to delay the disease progression. It has a potential disease-modifying effect. The uses include improving motor dysfunction, cognitive degeneration, neuroinflammation and mitochondrial dysfunction, and may regulate gut microbiota and the health of the central nervous system through the gut-brain axis mechanism. It is an inventive nutritional intervention solution with clinical application potential.

In one embodiment, the probiotic includes, but is not limited to, any species of *Lactobacillus, Bifidobacterium, Streptococcus, Lactococcus, Pediococcus,* and *Enterococcus,* or mixtures thereof; the bacterium includes, but is not limited to, a viable bacterium, a dead bacterium, a bacterium sterilized at low temperatures, a lysate, an extracellular polysaccharide, or a bacterial supernatant. All of the above-mentioned probiotics may affect neurological functions. The *Lactobacillus reuteri* strain Y7 used in the example of the present invention is a preferred embodiment.

In one embodiment, the essential amino acids are selected from leucine, isoleucine, valine, lysine, methionine, phenylalanine, tryptophan, threonine and histidine, which can be used alone or in combination according to different functional requirements.

In one embodiment, the ameliorated motor symptoms may include tremor, rigidity, bradykinesia, abnormal gait, dull facial expression, eyelid dysfunction, abnormal involuntary movements, etc.; non-motor symptoms may include anxiety, depression, sleep disorders, abnormal gastrointestinal motility, and dry eyes.

In one embodiment, the composition can be prepared into a pharmaceutical composition, a nutritional supplement, or a health food, and prepared with a proper pharmaceutically acceptable carrier, excipient or diluent into the form of a powder, a tablet, a capsule or a beverage to facilitate clinical application and long-term intake.

Example 1: Improvement effects of probiotic mixture formula on Parkinson's disease model rats

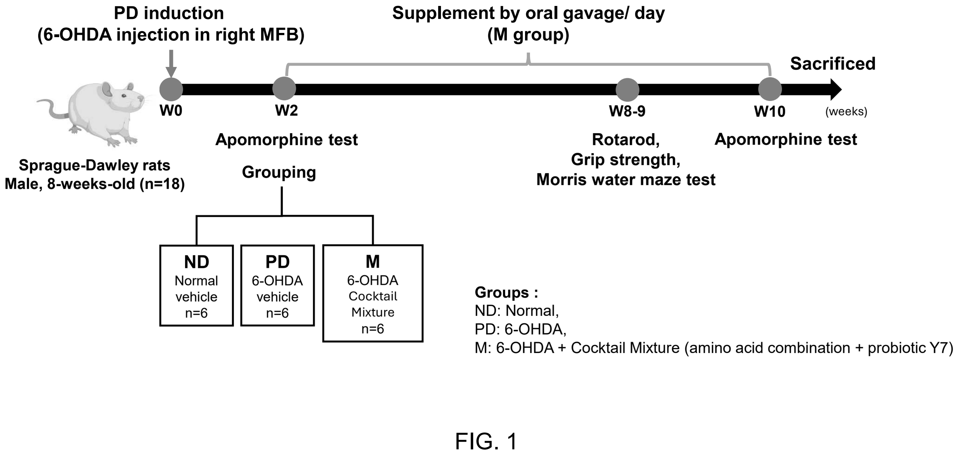

This example is intended to investigate the effects of a probiotic mixture formula comprising a specific probiotic *(Lactobacillus reuteri* Y7) and a functional amino acid combination (hereinafter referred to as the "TMU-01 formula") on the progression of Parkinson's disease. The experiment adopted a typical method of preparing a PD model to establish a 6-OHDA-induced rat model of Parkinson's disease, as shown in FIG. 1, in which the neurotoxin 6-hydroxydopamine (6-OHDA) was injected unilaterally into the medial forebrain bundle (MFB) of rats to induce dopaminergic neuron damage.

### I. Animal Grouping and Treatment

A total of 18 Sprague-Dawley rats were randomly assigned into three groups (n=6 in each group):
- Group ND: A normal control group, received normal saline gavage but no 6-OHDA injection;
- Group PD: A model group of Parkinson's disease, injected with 6-OHDA on the right MFB and then received normal saline gavage;
- Group M: A treatment group, modeled in the same way as Group PD, and orally administered with the TMU-01 formula daily.

The time-course of the experiment is as follows (as shown in FIG. 1):
- 6-OHDA injection was performed at week 0;
- Apomorphine-induced rotation tests were performed at weeks 2 and 10;
- Rotarod tests, grip strength tests, and Morris water maze tests were performed at weeks 8 and 9;
- The animals were sacrificed at week 10 for tissue staining and subsequent analysis.

### II. Assessment of Behavioral Function

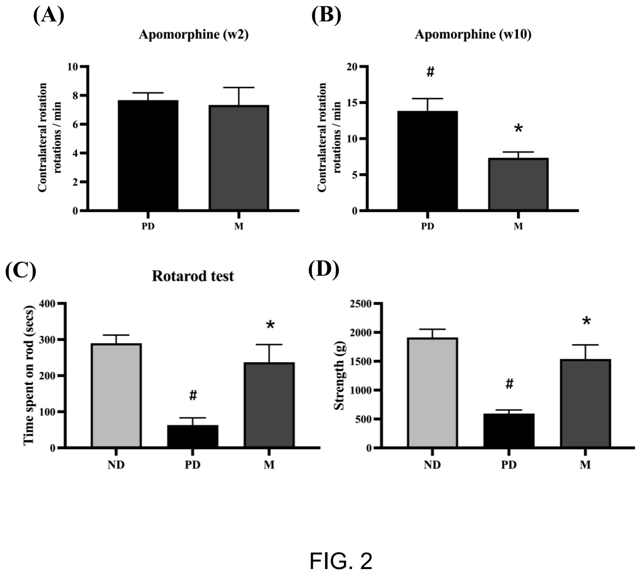

Apomorphine-induced rotational behavior tests were performed at weeks 2 and 10. The results showed that Group PD had significantly higher rotation times than Group ND in both tests (as shown in FIGS. 2A and 2B), indicating significant motor impairment. The number of rotations in Group M was significantly lower than that of Group PD, indicating that the formula can effectively improves abnormal rotational behavior.

In the rotarod balance test, the time that rats of Group PD spent on the platform was significantly shortened, indicating decreased motor coordination. Group M far outperformed Group PD in this indicator (as shown in FIG. 2C), indicating that the movement stability and balance ability have recovered to some degree.

Overall behavioral assessment results showed that the formula significantly improves motor impairments in the 6-OHDA-induced rat model of Parkinson's disease, including reducing rotational behavior as well as improving motor coordination and muscle strength performance, indicating it potentially benefits neuroprotection and motor function recovery.

### III. Assessment of Cognitive Function

To evaluate the effects of the formula on cognitive function, the Morris water maze test was performed at weeks 8 and 9 to assess spatial learning and memory abilities.

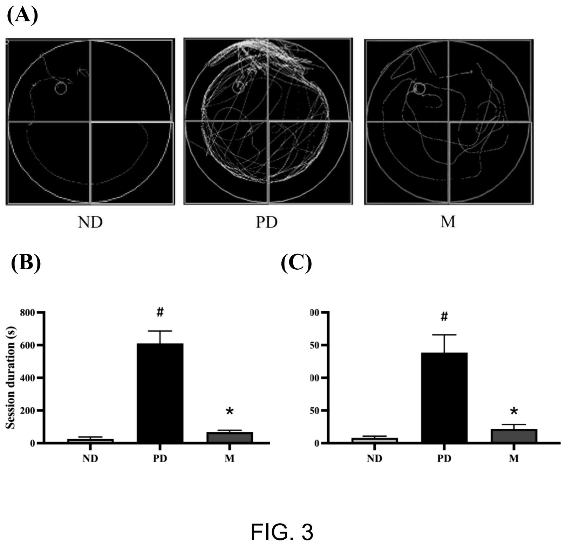

As shown in FIGS. 3A to 3C, rats in Group PD exhibited significantly prolonged escape latency, significantly decreased duration time in the target quadrant, and increased total swimming distance, indicating significant impairment in spatial learning and memory recall.

In contrast, Group M rats showed significantly better indicators than Group PD in terms of escape latency and duration time in the target zone. The formula can effectively improve spatial learning and memory abilities caused by Parkinson's disease. The cognitive performance of Group M was close to that of the ND group, indicating that the formula can improve cognitive dysfunction caused by Parkinson's disease.

Overall, the Morris water maze results showed that the formula has a significant protective effect on the spatial learning and memory abilities of the 6-OHDA-induced PD model, helping to delay Parkinson's disease-related cognitive decline.

### IV. Protective effect on neurons

To evaluate the protective effect of the formula on dopaminergic neurons in the substantia nigra of the midbrain, animals were sacrificed at week 10 and brain samples were collected for tyrosine hydroxylase (TH) immunohistochemical staining. The distribution and number of TH-positive neurons in the substantia nigra pars compacta (SNpc) were analyzed.

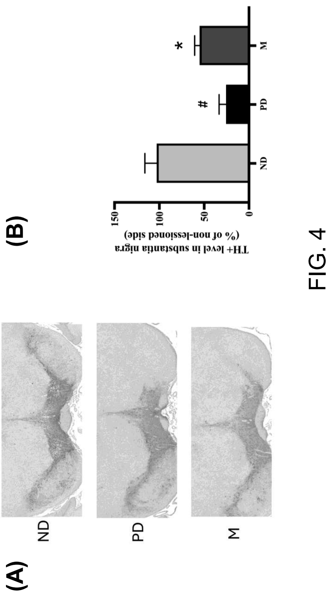

As shown in FIGS. 4A and 4B, the number of TH-positive cells in the SNpc region of rats in Group PD was significantly lower than that of Group ND, indicating a loss of dopaminergic neurons, which is in line with the neuropathological characteristics of Parkinson's disease.

The number of TH-positive cells in rats of Group M was significantly retained, and significantly more than that of Group PD. It is indicated that the formula intervention can effectively inhibit the damage and loss of neurons, demonstrating a protective effect on dopamine neurons.

These results demonstrate that, in addition to improving behavioral and cognitive performance, the formula also significantly preserves dopamine neurons at the tissue level, and that a significant increase in the number of TH-positive cells, indicating that the formula possesses neuroprotective effects and has the potential to delay the pathological progression of Parkinson's disease.

### V. Analysis of Inflammatory and Antioxidant Indicators

To further investigate the regulatory effects of the formula on neuroinflammation and oxidative stress, striatal tissue was collected from rats at week 10 for the analysis of mRNA expression of pro-inflammatory and antioxidant genes.

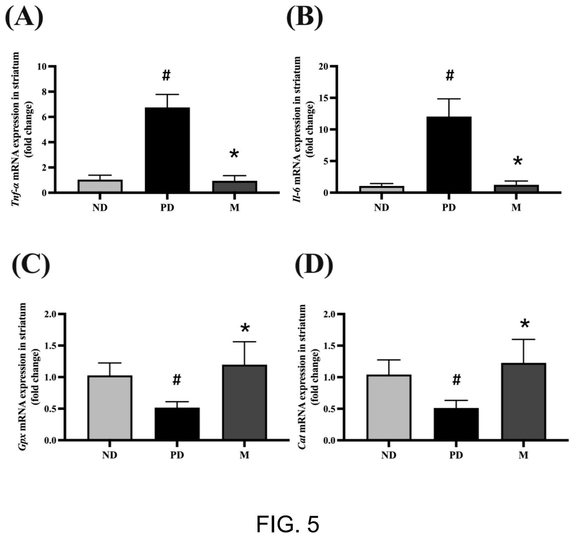

As shown in FIGS. 5A and 5B, compared to the normal control group ND, rats in Group PD showed a significant increase in the inflammatory factors Tumor Necrosis Factor-α (*Tnf-α*) and Interleukin-6 *(IL-6),* indicating increased neuroinflammatory responses in the model of Parkinson's disease. To the contrary, the expression levels of inflammatory factors *Tnf-α* and *IL-6* in the rats of Group M were significantly lower than those in Group PD, indicating that the formula has a significant anti-inflammatory effect.

As shown in FIGS. 5C and 5D, the expression levels of the antioxidant enzymes glutathione peroxidase *(Gpx)* and catalase *(Cat)* in Group PD were significantly lower than those of Group ND, indicating that the antioxidant system was damaged. The expression of *Gpx* and *Cat* genes in rats of Group M was significantly higher than that of Group PD, indicating that the formula can enhance the antioxidant capacity of cells and strengthen the defense mechanism of nerve cells against oxidative stress.

By combining the above results, the formula can simultaneously reduce the expression of neuroinflammatory factors and enhance the activity of antioxidant enzyme genes. It has the dual benefits of regulating neuroinflammation and oxidative stress, helping to protect neurons and delay neurodegeneration.

### VI. Analysis of Mitochondrial Function

To evaluate the effects of the formula on mitochondrial function, rat striatum and soleus muscle tissue were taken for the analysis of metabolic function-related indicators, including oxygen consumption rate (OCR) and extracellular acidification rate (ECAR), as well as the expression levels of mitochondrial biogenesis-related genes Sirtuin 1 (*Sirt1*) and Peroxisome proliferator-activated receptor gamma coactivator 1-alpha (*Pgc-1α*).

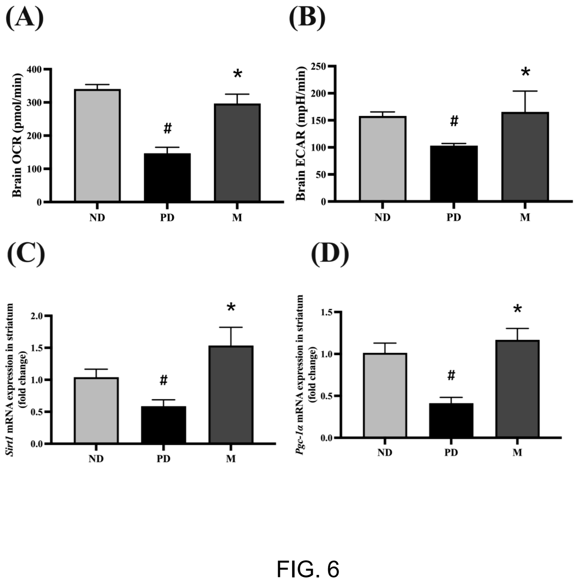

As shown in FIGS. 6A and 6B, the OCR and ECAR in the striatum of rats in Group PD were significantly lower than those of Group ND, indicating mitochondrial dysfunction. Mitochondrial function in Group M was significantly restored, with both OCR and ECAR significantly higher than those of Group PD, indicating significant improvement in mitochondrial function.

Further analysis of mitochondrial synthesis-related genes showed that the expression levels of *Sirt1* and *Pgc-1α* were significantly decreased in Group PD; while the expression levels in rats of Group M significantly increased (as shown in FIGS. 6C and 6D), further supporting the restoration and enhancement of mitochondrial function.

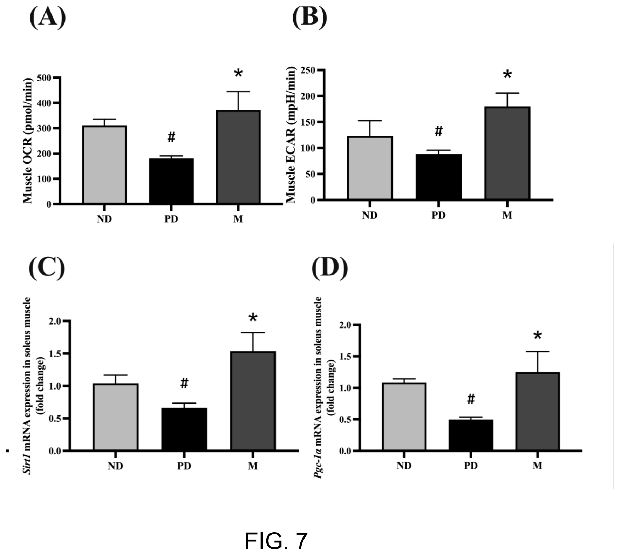

In addition, similar results were observed in muscle tissue. As shown in FIGS. 7A to 7D, in the soleus muscle, the expression of OCR, ECAR, *Sirt1,* and *Pgc-1α* decreased in Group PD, while significantly increased in Group M. It is indicated that the formula not only acts on the central nervous system but also has a restorative effect on mitochondrial function in peripheral tissues. The formula can improve mitochondrial health systemically.

In summary, the formula can effectively improve neurological impairment caused by energy deficits in animal models of Parkinson's disease by restoring mitochondrial energy metabolism and enhancing mitochondrial biogenesis, and has the potential for systemic mitochondrial regulation.

### VII. Analysis of Gut Microbiota

To evaluate the effect of the formula on regulating gut microbiota composition, fecal samples were collected from rats at week 10 of the experiment and analyzed using 16S rRNA high-throughput sequencing. The relative abundance analysis of microbiota was compared with the LEfSe statistical method.

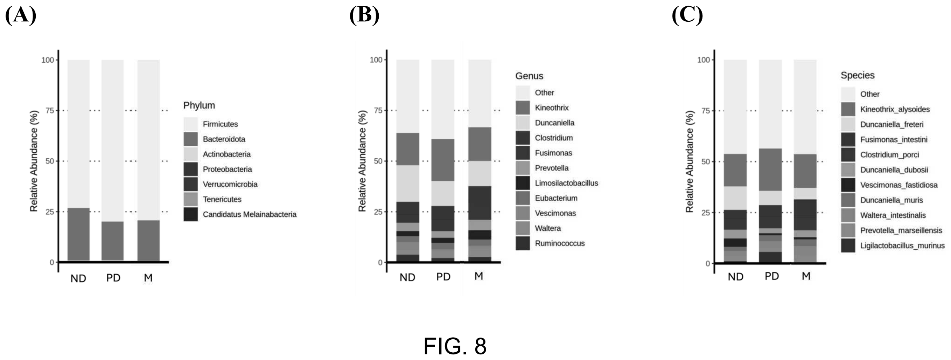

As shown in FIGS. 8A to 8C, at the phylum, genus, and species levels, the gut microbiota composition of rats in Group PD was significantly altered, the relative abundance of probiotic bacteria decreased and an increase in multiple potential pathogens was observed, indicating that the PD model leads to an imbalance in the gut microbiota.

The gut microbiota composition of rats in Group M was significantly different from that of Group PD, with a restoration effect of the relative abundance of probiotics and an increase in microbial diversity. The microbiome distribution of rats in Group M was close to that of Group ND, indicating that the formula can restore the damaged gut microbiota in the model of Parkinson's disease.

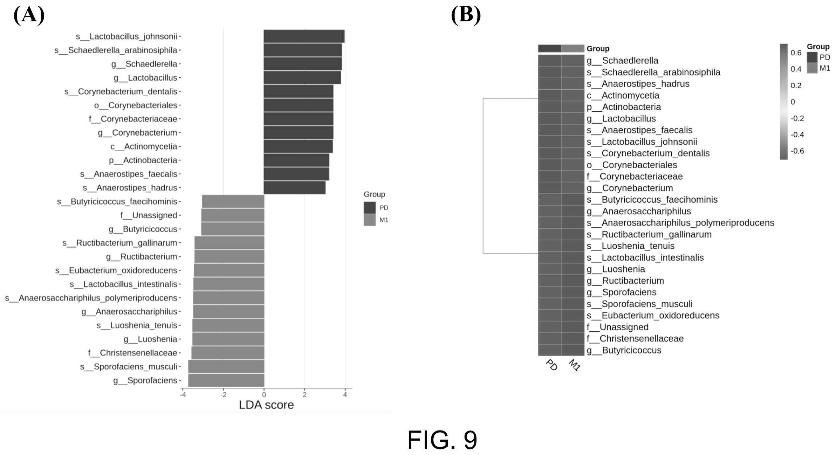

Furthermore, as shown in FIGS. 9A and 9B, by analyzing and comparing the differences in gut microbiota between the three groups via LEfSe, it is found that Group M has distinct microbial characteristics from Group PD. It is indicated that the intervention of the formula can lead to an increase in the dominance of specific beneficial bacteria species, has a good regulatory effect on the gut microbiota composition, and can restore the diversity and ecological balance of gut microbiota.

These results show that the TMU-01 formula has a favorable function of regulating gut microbiota, can restore the diversity and ecological balance of gut microbiota, and in turn may indirectly alleviate Parkinson's disease-related neurodegenerative symptoms through mechanisms involving the gut-brain axis. The formula provides potential benefits of systemic regulation of a disease.

Combined with the results of the examples of the present invention, it is demonstrated that the TMU-01 formula (a cocktail medical food composition comprising probiotics and a functional amino acid combination) can exhibit a plurality of significant physiological improvement effects in a 6-OHDA-induced animal model of Parkinson's disease, and has the following technical benefits:
1. Improving motor dysfunction: It effectively reduced the apomorphine-induced rotational behavior, increased the time spent on the rotarod platform, and improved grip strength test performance, indicating the ability to restore motor function in model rats of PD (as shown in FIG. 2).
2. Enhancing cognitive function: It improved spatial learning and memory performance in the Morris water maze test, shortened escape latency, and increased the time spent in the target zone, indicating a protective effect on spatial memory (as shown in FIG. 3).
3. Protecting dopamine neurons: The number of TH-positive cells in the SNpc region significantly increased, indicating that the formula has a neuroprotective effect and can delay the degeneration of dopamine neurons (as shown in FIG. 4).
4. Enhancing anti-inflammation and antioxidant capacity: It reduced the expression of inflammatory factors *Tnf-α* and *IL-6,* and increased the expression of antioxidant-related genes *Gpx* and *Cat,* helping to stabilize the neural microenvironment (as shown in FIG. 5).
5. Restoring mitochondrial metabolic function: It increased OCR and ECAR values in the striatum and soleus muscle, and promoted the expression of the mitochondrial biogenesis genes *Sirt1* and *Pgc-1α,* indicating the effects on restoring mitochondrial function and enhancing energy metabolism (as shown in FIGS. 6 and 7).
6. Regulating gut Microbiota and restoring microecological balance: It significantly increased the relative abundance of probiotics, improved the gut microbiota imbalance associated with Parkinson's disease, and determined significant differences in microbial indicators through LEfSe analysis (as shown in FIGS. 8 and 9).

The above results demonstrate that this formula involves multiple regulatory mechanisms, can simultaneously act on the central nervous system and peripheral tissues, and has significant improvement effects on behavioral function, neuropathology, molecular markers, and systemic metabolism in Parkinson's disease animal models. The formula is a promising adjuvant nutritional therapy with the potential to delay the disease progression, is used in preventive healthcare, early intervention, or disease regulation for Parkinson's disease, and has high clinical and industrial application values.

The inventiveness of the present invention lies in providing a medical food composition that combines probiotics and functional amino acid combination to specifically regulate key components associated with the progression of Parkinson's disease, including neurodegeneration, inflammatory responses, mitochondrial dysfunction, and cognitive decline, etc. This combination, with its multiple mechanisms of action and complementary functions, significantly outperforms existing products comprising only a single ingredient, allowing for delaying the progression of PD pathology from the source. The present invention takes Parkinson's disease as a clear indication and performs systematic validation using a 6-OHDA-induced Parkinson's disease animal model, covering multi-level indicators including motor behavior, cognitive performance, dopamine neuron integrity, inflammatory and antioxidant markers, mitochondrial function, and gut microbiota composition. The experimental results demonstrate that the composition can significantly improve motor and memory functions in animal models of Parkinson's disease, protects dopaminergic neurons, reduce neuroinflammation, enhance antioxidant capacity, restore mitochondrial metabolic activity in the brain and muscle tissue, and restore gut microbiota dominated by beneficial bacteria. These efficacies are closely correlated with the progression of Parkinson's disease, indicating that this formula is a non-drug nutritional intervention strategy with disease-modifying potential. Compared to conventional medicaments that merely alleviate symptoms, the medical food composition of the present invention can serve as a long-term, supportive, disease-modifying intervention, suitable for daily nutritional supplementation for patients with early-stage Parkinson's disease or groups at high risk. Furthermore, the composition can be prepared into a powder, a tablet, a capsule, or a beverage, facilitating long-term intake and clinical introduction. Besides serving as an adjuvant treatment option for Parkinson's disease patients, it is also applicable to preventive nutritional supplementation in the general population, and thus has high clinical translational value and potential for industrial application.

Accordingly, it is to be understood that the embodiments of the invention herein described are merely illustrative of the application of the principles of the invention. Reference herein to details of the illustrated embodiments is not intended to limit the scope of the claims, which themselves recite those features regarded as essential to the invention.

### BIOLOGICAL MATERIAL DEPOSIT

The *Lactobacillus reuteri* Y7 has been deposited in the Bioresource Collection and Research Center (BCRC) of Taiwan, deposited on November 29, 2022 under the accession number BCRC 911162. The strain has also been deposited in the International Patent Organism Depositary, National Institute of Technology and Evaluation (IPOD, NITE) (#120, 2-5-8 Kazusakamatari, Kisarazu-shi, Chiba 292-0818, Japan), deposited on February 9, 2024 under the accession number NITE BP-04078.

## Claims

1. A composition comprising (a) at least one probiotic with neuroprotective or metabolic regulation functions; and (b) a functional amino acid combination comprising four or more essential amino acids.

2. The composition according to claim 1, wherein the probiotic is a viable bacterium, a dead bacterium, a bacterium sterilized at low temperatures, a lysate, a bacterial supernatant, a cell wall extract, an extracellular polysaccharide or an immunostimulatory component thereof.

3. The composition according to claim 1, wherein the composition is a pharmaceutical composition, a nutritional supplement or a healthcare food.

4. The composition according to claim 1, wherein the composition comprises a pharmaceutically acceptable carrier, excipient, or diluent.

5. The composition according to claim 1, wherein the composition is in the form of a powder, a tablet, a capsule or a beverage.

6. A composition according to claim 1 for use in a medicament for delaying the progression of Parkinson's disease.

7. The composition according to claim 6, wherein delaying the progression of Parkinson's disease is to reduce Parkinson's disease-related motor symptoms or/and to reduce Parkinson's disease-related non-motor symptoms.

8. The composition according to claim 7, wherein the Parkinson's disease-related non-motor symptoms that are reduced include cognitive dysfunction, memory impairment, disorientation, mental decline, executive function impairment, irritability, changes in interpersonal behavior, anxiety, mood swings, depression, apathy, sleep disorders, dysautonomia, olfactory disorder, abnormal pain perception, or fatigue.

9. The composition according to claim 8, wherein the dysautonomia includes constipation, hypotension, urinary dysfunction, or abnormal sweating.

10. The composition according to claim 9, wherein the Parkinson's disease-related motor symptoms that are reduced include tremor, muscle rigidity, bradykinesia, unsteady gait, foot shuffling, abnormal involuntary movements, facial expression disorders, and ocular dysfunction.

11. The composition according to claim 10, wherein the abnormal involuntary movements include dyskinesia and dystonia.

12. The composition according to claim 10, wherein the facial expression disorders include mask-like facies and facial paralysis.

13. The composition according to claim 10, wherein the ocular dysfunction includes apraxia of eyelid opening, blepharospasm, reduction of tear film secretion, and evaporative dry eye caused by non-blinking responses.

14. The composition according to claim 6, wherein delaying the progression of Parkinson's disease is achieved by enhancing anti-inflammatory capacity, enhancing antioxidant capacity, restoring mitochondrial metabolic function, regulating gut microbiota, or restoring intestinal microecological balance.

15. The composition according to claim 7, wherein the composition is used in daily nutritional supplementation for Parkinson's disease patients or Parkinson's disease high risk groups to delay the disease progression.
